# EUROPEAN PATENT SPECIFICATION

(11) **EP 1 894 545 B1**
(45) Date of publication and mention of the grant of the patent: **27.04.2011**
(21) Application number: 07253073.6
(22) Date of filing: 03.08.2007
(51) Int. Cl.: A61F 2/84

(54) **Multiple in vivo implant delivery device**
Einführvorrichtung für mehrere In-vivo-Implantate
Dispositif de mise en place d'implants multiples in vivo

(30) Priority: 28.08.2006 US 511097
(43) Date of publication of application: 05.03.2008
(73) Proprietor: Cordis Corporation, Miami Lakes, FL 33014 (US)
(72) Inventor: Davila, Luis A., Alpharetta, GA 30005 (US); Dwyer, Clifford J., Weston, FL 33326 (US)
(74) Representative: Belcher, Simon James

(56) References cited:
- EP-A- 1 258 230
- WO-A-2006/026371
- US-A- 5 817 101

## Description

The present invention relates to the field of medical devices, and more particularly to a device for the delivery of multiple *in vivo* implant devices.

Vascular disease is a leading cause of premature mortality in developed nations, often presenting as a vascular aneurysm. A vascular aneurysm is a localized dilation of a vessel wall, due to thinning or weakness of the wall structure, or separation between layers of the vessel wall. If untreated, the aneurysm may burst and hemorrhage uncontrollably. Aneurysms are particularly dangerous and prevalent in the aorta, because the aorta supplies blood to all other areas of the body, and because the aorta is subject to particularly high pressures and stresses accordingly. Rupture of an aortic aneurysm is the 15^{th} leading cause of death the United States, afflicting 5% of older men.

It is known to treat vascular aneurysms surgically where blood pressure control medication is unsuccessful at arresting growth of the aneurysm. Surgery often involves the insertion of a vascular stent graft to exclude the aneurysm and carry blood past the dilated portion of the vessel, relieving the pressure on the aneurysm. Other applications for the treatment of vascular disease is the use of a stent to open an occluded vessel, commonly a coronary artery. Such stents may include a coating designed to release a pharmaceutical compound into the bloodstream at a controlled rate.

Moreover, it would be advantageous to design a stent graft that is collapsible to facilitate percutaneous insertion by minimally invasive surgical techniques. Additionally, percutaneous insertion requires the design and development of a delivery apparatus that can effectively position and deploy the vascular stent.

It is often advantageous to provide multiple stents to support a vessel. For example, a vessel may be subject to multiple blockages along its length. Alternately, it often enhances the flexibility of the stent and improves stent placement accuracy to provide plural serial stents rather than a single stent of equivalent length. Therefore, an efficient delivery apparatus for plural *in vivo* implant devices, such as prosthetic stents, is desired in the art.

WO-A-2006/026371 relates to a system for placing multiple expandable medical devices within a body vessel and shows all the features of the preamble of claim 1. In one embodiment two prosthetic valves are provided on mounting regions either side of an intermediate member.

Therefore, in order to overcome these and other deficiencies in the prior art, provided according to the present invention is an apparatus for the delivery of plurality of in vivo implant devices, for example, segmented vascular stents, as defined in appended claim 1.

These and other features, benefits, and advantages of the present invention will be made apparent with reference to the following detailed description, appended claims, and accompanying figures, wherein like reference numerals refer to like structures across the several views.

Embodiments of the invention will now be described by way of example with reference to the accompanying drawings, in which:
Figure 1 illustrates a schematic version of a delivery catheter useful for understanding the invention.
Figure 2 illustrates a distal end of the delivery catheter of Figure 1.
Figure 2A illustrates a distal portion of the delivery catheter after a first distal stent has been deployed useful for understanding the invention.
Figure 3 illustrates a cross-sectional view of a distal end of a delivery catheter according to Figure 1.
Figure 4 illustrates an alternative delivery catheter useful for understanding the invention.
Figure 5 illustrates an alternative embodiment of a delivery catheter according to the present invention.
Figure 6 illustrates another delivery catheter useful for understanding the invention.
Figure 7 illustrates a second embodiment of a delivery catheter according to the present invention.
Figure 8 illustrates a further delivery catheter useful for understanding the invention.
Figure 9 illustrates a third embodiment of a delivery catheter according to the present invention.

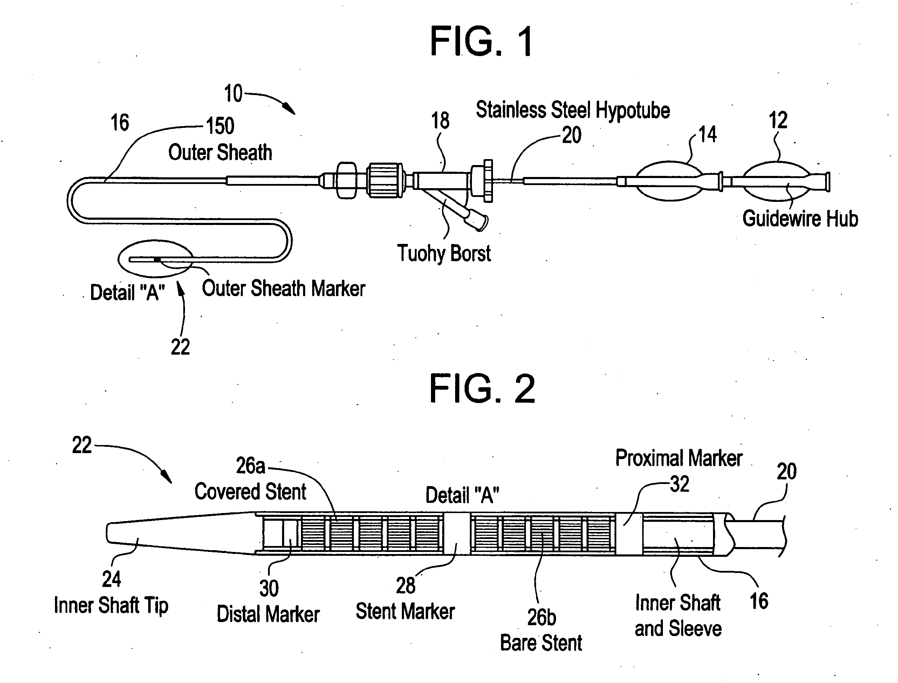

Referring now to Fig. 1, illustrated schematically is a delivery catheter, generally 10. Delivery catheter 10 has a first handle 12 at a proximal end in communication with a guide wire (not shown) extending axially through the delivery catheter 10. A second handle 14 is in communication with an inner shaft 20 of the delivery catheter 10. An insertion valve 18, preferably a tuohy-borst valve, is in communication with an outer sheath 16 of the delivery catheter 10. Each of the guide wire, the inner shaft 20, and outer sheath 16 are independently axially displaceable relative to one another, by movement of first and second handles 12, 14, with respect to one another and/or to the insertion valve 18.

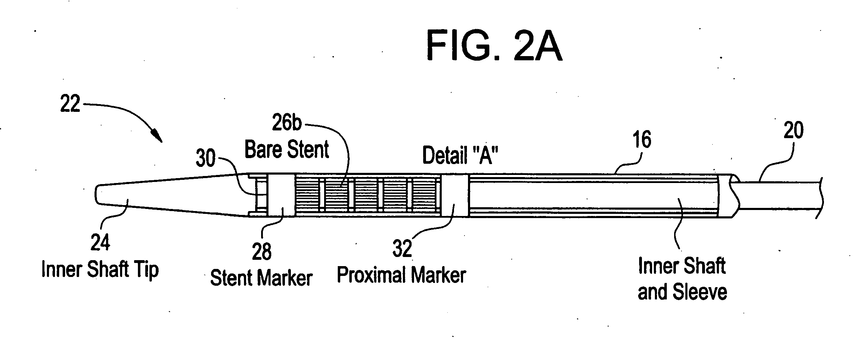

Referring now to Fig. 2, illustrated is a distal end, generally 22, of the delivery catheter 10 for Fig. 1. Inner shaft 20 ends at a distal tip 24. Outer sheath 18 is preferably connected with the distal tip 24, to enable the delivery catheter 10 to rotate as a unit upon insertion and/or to present a continuous outer surface of the delivery catheter 10, thereby reducing resistance to insertion. One or more prosthetic stents 26 are crimped, or reduced in diameter from a fully deployed diameter, onto inner shaft 20 near the distal tip 24. A radiopaque stent marker 28 surrounds the inner shaft 20 between adjacent stents 26a, 26b, thereby radiographically locating, for example by fluoroscopy, the one or more stents 26a, 26b for *in vivo* delivery and implantation. Additionally, the delivery catheter 10 may include distal radiopaque marker 30 that moves together with the distal tip 24, and proximal radiopaque marker 32 that is secured in axial position along the inner shaft 20. The stents 26 are preferably self-expanding, and/or comprise a shape memory material, comprising Nitinol or some alloy thereof. In this exemplary embodiment, stent 26a is a covered stent, or one having a coating to, for example, enhance biocompatibility and/or to elute a pharmaceutical compound into the body. By contrast, stent 26b is an uncovered or bare stent. Once positioned as desired by the surgeon, the stents 26a, 26b are deployed individually, by withdrawing the outer sheath 16 from over the stents 26a, 26b, which are then free to expend in the vascular environment.

Preferably, the stents 26a, 26b are deployable individually, and only one stent need be deployed at a time. Referring now to Fig. 2A, after a first distal stent is deployed, in this case covered stent 26a, the distal tip 24 may be withdrawn to meet the outer sheath 16.

Stent marker 28 is preferably free-floating over inner shaft 20. Therefore as the distal tip 24 is withdrawn proximally, the stent marker 28 is positioned adjacent a distal end of outer sheath 16, approximately co-located with distal marker 30. Proximal marker 32 having been advanced distally relative to the outer sheath 16 with the deployment of the first distal stent, i.e., covered stent 26a, said proximal marker 32 remains immediately proximal to the bare stant 26b.

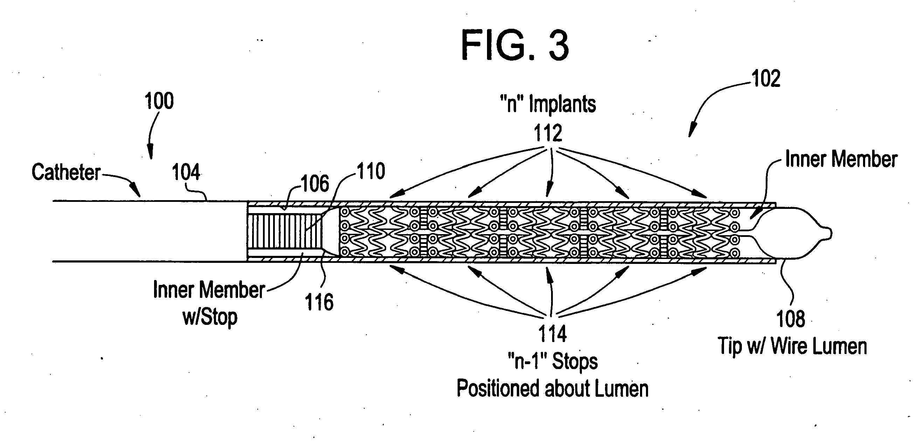

Turning then to Fig. 3, illustrated schematically in partial cross-section is a delivery catheter, generally 100, and more specifically a distal end thereof, generally 102. An outer sheath 104 has an axial lumen 106 therethrough, and extends to a distal tip 108. Outer sheath 104 is preferably connected with the distal tip 108, to enable the delivery catheter 100 to rotate as a unit upon insertion and/or to present a continuous outer surface of the delivery catheter 100, thereby reducing resistance to insertion. Inner shaft 110 extents through axial lumen 106 to terminate at the distal tip 108. Preferably, inner shaft 110 has an axial lumen (not shown) running therethrough, to admit a guide wire to assist in inserting the delivery catheter 100.

A plurality of implants 112, for example vascular or other stents, are crimped, i.e., reduced in diameter from a fully deployed diameter to fit within axial lumen 106 for insertion and delivery, to the inner shaft 110. Between each implant 112 is an axial stop 114, which are either free-floating, i.e., axially displaceable over the inner shaft 110 within the axial lumen 106, or secured to the inner shaft 110. A proximal stop 116 is secured to the inner shaft 110 proximally from all implants 112, and limits the axial motion of implants 112 and/or stops 114 between itself and the distal tip 108.

According to the present invention, the stops 114 vary in width radially and optionally also axially to counteract the stored compressive energy in the delivery catheter 100 during the deployment of successive implants 112. Accordingly, the deployment force required of each implant 112 is more uniform over the course of the procedure, which assists in the more precise and accurate control of deployed position.

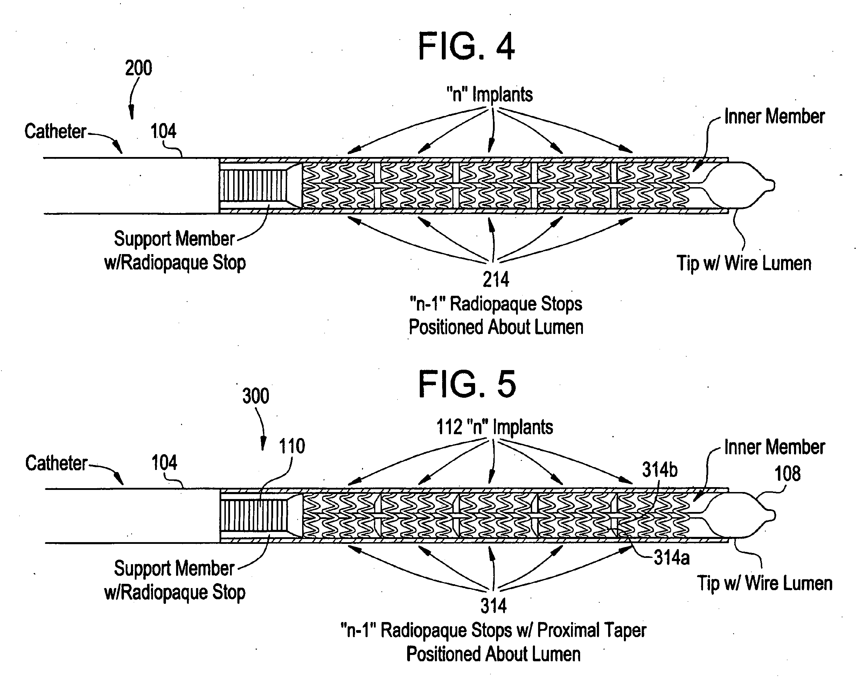

Referring now to Fig. 4, illustrated is an alternate delivery catheter, generally 200. The stops 214 between each of the implants 112 are radiopaque. The radiopaque stops 214 assist in by radiographically locating, for example by fluoroscopy, the one or more implants 114 for precise control of deployment.

Referring now to Fig. 5, illustrated is an embodiment of a delivery catheter, generally 300. In this embodiment, the stops 314 between each of the implants 112 are proximally tapered, i.e., they are tapered from a narrower diameter on a proximal side 314a to a wider diameter on a distal side 314b. The proximally tapered stops 314 assist may be radiopaque or non-radiopaque.

It is known that self-expanding implants, such as implants 114 have a tendency to 'jump', or to move axially from the open end of the outer sheath 104 in the process of expanding to their deployed diameter, as the outer sheath 104 is retracted. The tapered stops 314 assist in the deployment of implants 114 because they allow the implant to pass over the stop 314 with a reduced risk of catching the implant 114 on the stop 314. Therefore, the implant 114 is deployed more consistently without unexpected catching, improving the precision and accuracy of the deployment position.

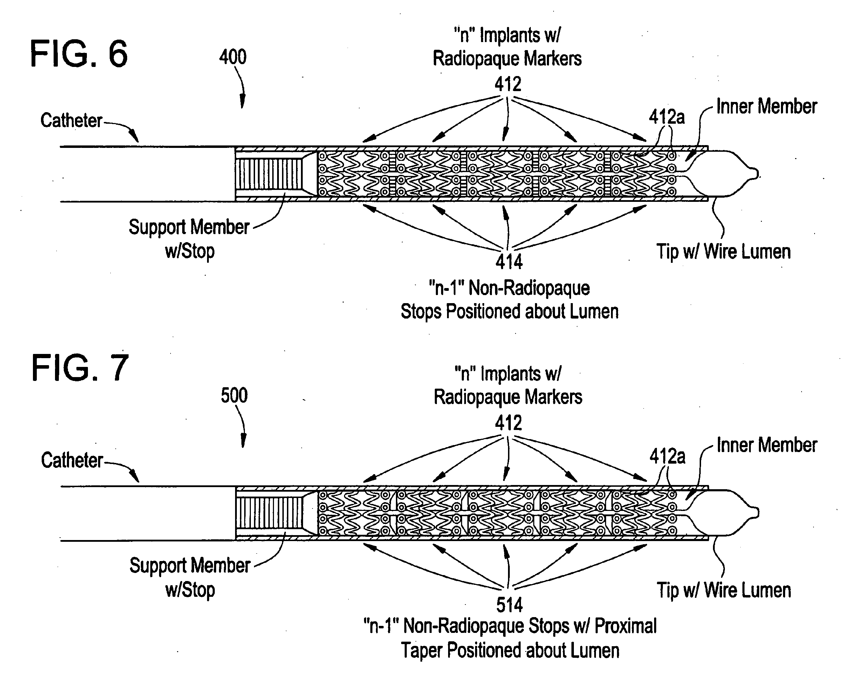

Referring now to Fig. 6, illustrated is an alternate delivery catheter, generally 400. Implants 412 are provided with integral radiopaque markers 412a, preferably more than one, and preferably distributed over the circumference of the implant 412 at both the proximal and distal ends thereof. In this embodiment, stops 414 are non-radiopaque, to avoid interference with the imaging of the radiopaque markers 412a on the implants 412 themselves.

Referring now to Fig. 7, illustrated is an alternate embodiment of a delivery catheter, generally 500. In this embodiment, the stops 514 between each of the implants 112 are proximally tapered, as in the embodiment of Fig. 5. Moreover, the proximally tapered stops 514 are non-radiopaque. Implants 412 have radiopaque markers 412a, as described with reference to Fig. 6.

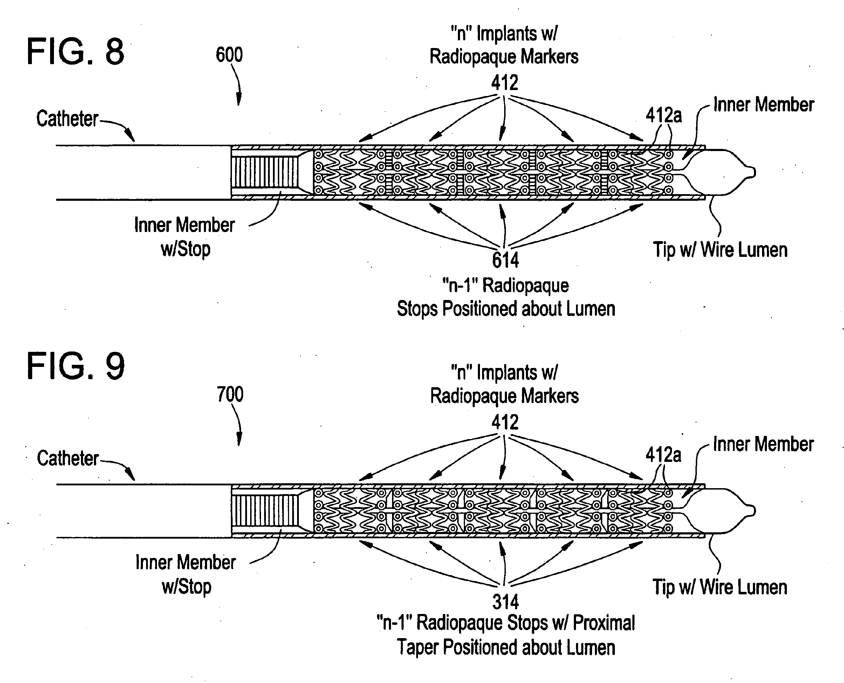

Referring now to Fig. 8, illustrated is an alternate delivery catheter, generally 600. The stops 614 between each of the implants 112 are of generally constant diameter. Moreover, the constant diameter stops 614 are radiopaque, as described with reference to the embodiment of Fig. 4. Implants 412 have radiopaque markers 412a, as described with reference to Fig. 6.

Referring now to Fig. 9, illustrated is an alternate embodiment of a delivery catheter, generally 700. In this embodiment, the stops 314 between each of the implants 412 are proximally tapered and radiopaque, as described with reference to Fig. 5. Implants 412 have radiopaque markers 412a, as described with reference to Fig. 6.

Accordingly, it will be appreciated from Figs. 5, 7 and 9 that the axial stops are tapered, preferably proximally tapered. Axial stops may be radiopaque or non-radiopaque. The implants themselves may or may not have radiopaque markers. Moreover, any of these features may be used or omitted in any permutation as desired.

The delivery catheter 100 having a central lumen (not shown) for a guide wire will by recognized by those skilled in the art as an over-the-wire type configuration. Alternately, however, the distal tip 108 of the delivery catheter 100 may include an abbreviated passage to accept the guide wire, as part of a so-called rapid-exchange design as is known in the art. Accordingly, the delivery catheter 100 need not be threaded over the entire length of the guide wire, and the guide wire can be shorter. Moreover, using a rapid-exchange design obviates the need for a central lumen to admit the guide wire through all or most of its length. Accordingly, the overall diameter of the delivery catheter can be advantageously reduced. Since the point of connection in the rapid-exchange design is distal of the implants 114, the guide wire would necessarily be outside the implants after deployment, to be subsequently withdrawn.

## Claims

1. A catheter (300, 500, 700) for the delivery of a plurality of *in vivo* implant devices, the catheter comprising:
an outer sheath (104) having an axial lumen (106) therethrough;
an inner shaft (110) extending from a proximal end of the catheter to a distal tip (108) through the axial lumen (106);
a proximal stop (116) secured to the inner shaft (110) and sized to prevent passage through the axial lumen (106); and
one or more axial stops (314, 315) along the length of the inner shaft (110) between the proximal stop (116) and the distal tip (108), at least one axial stop (314, 514) axially positioned between two adjacent implant devices (112, 412) and **characterised in that** at least one of the one or more axial stops (314, 514) is tapered in diameter along its length.

2. A catheter (300, 500, 700) according to claim 1, wherein the at least one of the one or more axial stops (314, 514) is tapered from a narrower diameter on a proximal side (314a) to a wider diameter on a distal side (314b).

3. The catheter (300, 500, 700) according to claim 1, wherein at least one of the one or more axial stops (314, 514) are fixed to the inner shaft (110).

4. The catheter (300, 500, 700) according to claim 1, wherein at least one of the one or more axial stops (314, 514) are axially slidable on the inner shaft (110).

5. The catheter (700) according to claim 1, wherein at least one of the one or more axial stops (314) is radiopaque.

6. The catheter (300, 500, 700) according to claim 1, wherein at least one of the one or more axial stops (314, 514) comprises a plurality of axial stops, and the axial stops vary in width.

7. The catheter (300, 500, 700) according to claim 6, wherein each of the plurality of axial stops (314, 514) is wider than a distally adjacent axial stop.

8. The catheter (300, 500, 700) according to claim 1, further comprising an axial lumen through at least a part of the inner shaft (110).

9. The catheter (300, 500, 700) according to claim 1, further comprising a passage at the distal tip (108) for admitting a guide wire, and a rapid exchange mechanism for attaching or detaching the delivery catheter from the guide wire.

10. The catheter (300, 500, 700) according to claim 1, wherein the outer sheath (104) is connected with the distal tip (108) to present a continuous outer surface of the delivery catheter.

11. The catheter (300, 500, 700) according to claim 1, further comprising a plurality of self-expanding *in vivo* implant devices (112, 412) radially between the inner shaft (110) and the outer sheath (104), proximally of the proximal stop (116), and having at least one of said one or more axial stops (314, 514) between any two implant devices (112, 412).

12. The catheter (500, 700) according to claim 11, wherein one or more of the implant devices (412) comprises one or more radiopaque markers (412a).

13. The catheter (300, 500, 700) according to claim 1, wherein the proximal stop (116) is radiopaque.

## Patentansprüche

1. Katheter (300, 500, 700) für die Zuführung mehrerer In-Vivo-Implantatvorrichtungen, wobei der Katheter Folgendes umfasst:
eine äußere Hülle (104) mit einem axialen Lumen (106) dort hindurch;
einen inneren Schaft (110), welcher sich durch das axiale Lumen (106) von einem proximalen Ende des Katheters zu einer distalen Spitze (108) erstreckt;
einen proximalen Anschlag (116), welcher an dem inneren Schaft (110) befestigt ist und dimensioniert ist, um einen Durchgang durch das axiale Lumen (106) zu verhindern; und
einen oder mehrere axiale Anschläge (314, 315) entlang der Länge des inneren Schaftes (110) zwischen dem proximalen Anschlag (116) und der distalen Spitze (108), wobei zumindest ein axialer Anschlag (314, 514) axial zwischen zwei benachbarten Implantatvorrichtungen (112, 412) positioniert ist und **dadurch gekennzeichnet, dass** zumindest einer der einen oder mehreren axialen Anschläge (314, 514) im Durchmesser entlang seiner Länge abgeschrägt ist.

2. Katheter (300, 500, 700) gemäß Anspruch 1, wobei der zumindest eine der einen oder mehreren axialen Anschläge (314, 514) von einem engeren Durchmesser auf einer proximalen Seite (314a) zu einem breiteren Durchmesser auf einer distalen Seite (314b) abgeschrägt ist.

3. Katheter (300, 500, 700) gemäß Anspruch 1, wobei zumindest einer der einen oder mehreren axialen Anschläge (314, 514) an dem inneren Schaft (110) fixiert ist.

4. Katheter (300, 500, 700) gemäß Anspruch 1, wobei zumindest einer der einen oder mehreren axialen Anschläge (314, 514) axial auf dem inneren Schaft (110) verschiebbar ist.

5. Katheter (700) gemäß Anspruch 1, wobei zumindest einer der einen oder mehreren axialen Anschläge (314) röntgenstrahlenundurchlässig ist.

6. Katheter (300, 500, 700) gemäß Anspruch 1, wobei zumindest einer der einen oder mehreren axialen Anschläge (314, 514) mehrere axiale Anschläge umfasst und die axialen Anschläge in einer Breite variieren.

7. Katheter (300, 500, 700) gemäß Anspruch 6, wobei jeder der mehreren axialen Anschläge (314, 514) breiter als ein distal benachbarter axialer Anschlag ist.

8. Katheter (300, 500, 700) gemäß Anspruch 1, welcher weiter ein axiales Lumen durch zumindest einen Teil des inneren Schaftes (110) umfasst.

9. Katheter (300, 500, 700) gemäß Anspruch 1, welcher weiter einen Durchgang an der distalen Spitze (108) zum Einlassen eines Führungsdrahtes und einen Schnelltauschmechanismus zum Anbringen oder Lösen des Zuführungskatheters von dem Führungsdraht umfasst.

10. Katheter (300, 500, 700) gemäß Anspruch 1, wobei die äußere Hülle (104) mit der distalen Spitze (108) verbunden ist, um eine durchgehende äußere Fläche des Zuführungskatheters zu präsentieren.

11. Katheter (300, 500, 700) gemäß Anspruch 1, welcher weiter radial zwischen dem inneren Schaft (110) und der äußeren Hülle (104), proximal zu dem proximalen Anschlag (116) mehrere selbstausdehndene In-Vivo-Implantatvorrichtungen (112, 412) umfasst und zumindest einen der einen oder mehreren Anschläge (314, 514) zwischen zwei Implantatvorrichtungen (112, 412) aufweist.

12. Katheter (500, 700) gemäß Anspruch 11, wobei eine oder mehrere der Implantatvorrichtungen (412) einen oder mehrere Röntgenmarker (412a) umfasst.

13. Katheter (300, 500, 700) gemäß Anspruch 1, wobei der proximale Anschlag (116) röntgenstrahlenundurchlässig ist.

## Revendications

1. Cathéter (300, 500, 700) de mise en place d'une pluralité de dispositifs d'implants *in vivo*, le cathéter comprenant :
> une gaine externe (104) ayant un lumen axial (106) dans celle-ci ;
> une gaine interne (110) s'étendant d'une extrémité proximale du cathéter à une pointe distale (108) dans le lumen axial (106) ;
> un arrêt proximal (116) fixé à la tige interne (110) et dimensionnée pour empêcher le passage dans le lumen axial (106) ; et
> un ou plusieurs arrêts axiaux (314, 315) sur la longueur de la gaine interne (110) entre l'arrêt proximal (116) et la pointe distale (108), au moins un arrêt axial (314, 514) étant positionné axialement entre deux dispositifs d'implants adjacents (112, 412) et **caractérisé en ce qu'**au moins l'un parmi un ou plusieurs arrêts axiaux (314, 514) a un diamètre conique sur sa longueur.

2. Cathéter (300, 500, 700) selon la revendication 1, dans lequel au moins l'un parmi un ou plusieurs arrêts axiaux (314, 514) évolue d'un diamètre plus étroit sur le côté proximal (314a) à un diamètre plus large sur un côté distal (314b).

3. Cathéter (300, 500, 700) selon la revendication 1, dans lequel au moins l'un parmi un ou plusieurs des arrêts axiaux (314, 514) est fixé à la tige interne (110).

4. Cathéter (300, 500, 700) selon la revendication 1, dans lequel au moins l'un parmi un ou plusieurs des arrêts axiaux (314, 514) peut coulisser axialement sur la tige interne (110).

5. Cathéter (700) selon la revendication 1, dans lequel au moins l'un parmi un ou plusieurs des arrêts axiaux (314) est radio-opaque.

6. Cathéter (300, 500, 700) selon la revendication 1, dans lequel au moins l'un parmi un ou plusieurs des arrêts axiaux (314, 514) comprend une pluralité d'arrêts axiaux et les arrêts axiaux varient en largeur.

7. Cathéter (300, 500, 700) selon la revendication 6, dans lequel chacun des multiples arrêts axiaux (314, 514) est plus large qu'un arrêt axial adjacent sur le plan distal.

8. Cathéter (300, 500, 700) selon la revendication 1, comprenant en outre un lumen axial dans au moins une partie de la tige interne (110).

9. Cathéter (300, 500, 700) selon la revendication 1, comprenant en outre un passage à la pointe distale (108) pour recevoir un fil-guide et un mécanisme d'échange rapide pour fixer ou détacher le cathéter de mise en place du fil-guide.

10. Cathéter (300, 500, 700) selon la revendication 1, dans lequel la gaine externe (104) est raccordée à la pointe distale (108) pour présenter une surface externe continue du cathéter de mise en place.

11. Cathéter (300, 500, 700) selon la revendication 1, comprenant en outre une pluralité de dispositifs d'implants *in vivo* auto-expansibles (112, 412) sur le plan radial entre la tige interne (110) et la gaine externe (104) sur le plan proximal de l'arrêt proximal (116) et ayant au moins l'un parmi lesdits un ou plusieurs arrêts axiaux (314, 514) entre deux dispositifs d'implants quelconque (112, 412).

12. Cathéter (500, 700) selon la revendication 11, dans lequel un ou plusieurs des dispositifs d'implants (412) comprennent un ou plusieurs marqueurs radio-opaques (412a) .

13. Cathéter (300, 500, 700) selon la revendication 1, dans lequel l'arrêt proximal (116) est radio-opaque.
